# EUROPEAN PATENT APPLICATION

(11) **EP 0 575 107 A1**
(43) Date of publication of application: **22.12.1993**
(21) Application number: 93304541.1
(22) Date of filing: 11.06.1993
(51) Int. Cl.: A61F 2/38

(54) **Elbow joint prosthesis**

(30) Priority: 19.06.1992 GB 9213110
(71) Applicant: HILA INC., Baltimore, Maryland (US)
(72) Inventor: Stossel, Clifford Alain, Headcorn, Ashford, Kent TN27 9RB (GB)
(74) Representative: Perry, Robert Edward

(57) **Abstract**

A joint prosthesis, for use in surgery, comprises two elongate members (1, 2) adapted to be fixed respectively to associated bones, e.g. to the humerus and to the ulna, the elongate members comprising complementary means (4, 5) for a hinged attachment (3) at respective ends thereof.

## Description

### Field of the Invention

This invention relates to a prosthesis adapted to provide a hinged replacement joint, particularly for the treatment of disease or injury to the elbow.

### Summary of the Invention

According to the present invention, a joint prosthesis comprises two elongate members adapted to be fixed respectively to associated bones, e.g. to the humerus and to the ulna, the elongate members comprising complementary means for a hinged attachment at their adjacent ends.

The novel prosthesis can be used to treat arthritis, degenerative or rheumatoid, and tumours, primary or secondary, especially of the elbow joint. For example, where injury has damaged or destroyed the elbow joint, and conservative treatment or reconstructive repair is not possible, the novel prosthesis can be used to replace the injured articulation.

### Description of the Invention

The invention will now be described by way of example only with reference to the accompanying drawings, and to the provision of an elbow prosthesis. In the drawings:

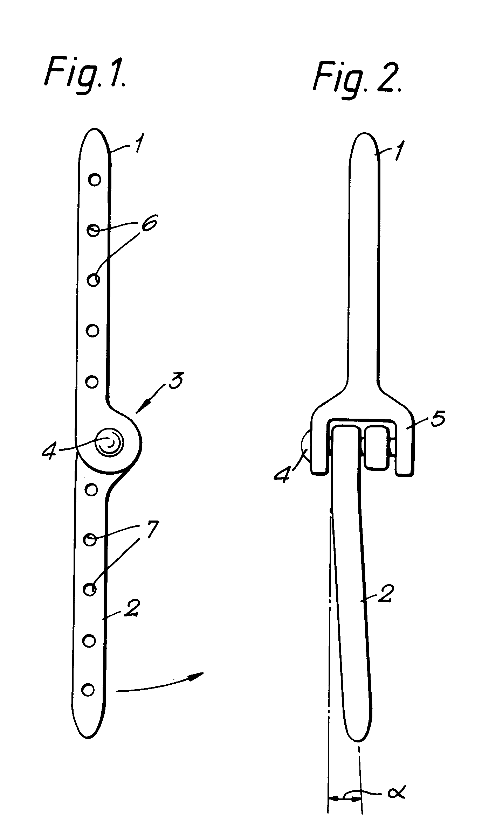

Figs. 1 and 2 are mutually-orthogonal side views of an elbow prosthesis embodying this invention.

The drawings show a prosthesis comprising two stem parts 1, 2 which are hinged together, the hinge 3 comprising a pin member 4 which is inserted, at the choice of the surgeon, from either side. The pinned hinge is one example of hinged attachments which can be used within the scope of this invention. Similarly, the provision of either stem part with a forked end, as shown at 5 for the humeral stem 1 in the drawing, is merely illustrative. The hinge 3 allows relative movement of the stem parts (co-linear as shown) in the direction of the arrow shown in Fig. 1 but usually not in the opposite sense.

The humeral component of an elbow prosthesis according to this invention can be fixed to the humeral shaft by interlocking screws which pass through holes 6 in the stem 1 of the prosthesis and the humeral bone. Several screws are used and, according to the length of the humeral stem, the prosthesis can be used to fix a fracture of the humeral shaft at the same time. The distal portion of the prosthesis fits into the ulna. It is fixed to the ulna by screws through holes 7 in the stem of the ulna component 2. This method of fixation allows a fracture of the shaft of the ulna to be fixed at the same time.

The stem of the ulna component of the prosthesis can either be placed in line with the stem of the humeral component or it can be offset by up to 10° into a valgus direction. An offset angle α is shown in Fig. 2.

The stems of the humeral and ulna components can be varied in length, from 50 to 300 mm, according to the needs of the individual patient. They are often of substantially the same length. The prosthesis can be made in various sizes suitable for the differing sizes of the elbows of individual patient.

The prosthesis can be made in titanium alloy, stainless steel, or another biocompatible material.

## Claims

1. A joint prosthesis, for use in surgery, comprising two elongate members (1, 2) adapted to be fixed respectively to associated bones, e.g. to the humerus and to the ulna, the elongate members comprising complementary means (4, 5) for a hinged attachment (3) at respective ends thereof.

2. A prosthesis according to claim 1, which additionally comprises a pin member (4) which can be inserted through respective complementary apertures in the elongate members, to provide the hinged attachment.

3. A prosthesis according to claim 1 or claim 2, wherein either or each elongate member is 50 to 300 mm long.

4. A prosthesis according to any preceding claim, wherein either or each elongate member comprises, along its length, a plurality of apertures (6, 7) adapted for screw-fixing to the associated bones.

5. A prosthesis according to any preceding claim, wherein the hinged attachment allows substantially no lateral displacement of one elongate member with respect to the other, or such lateral displacement by an angle (α) of no more than 10°.

6. A prosthesis according to any preceding claim, wherein the hinged attachment allows movement of the elongate members in one sense with respect to their co-linear position, but not the other.

7. A joint prosthesis substantially as herein described with reference to the accompanying drawings.
